# EUROPEAN PATENT APPLICATION

(11) **EP 3 778 902 A1**
(43) Date of publication of application: **17.02.2021**
(21) Application number: 19775872.5
(22) Date of filing: 28.03.2019
(51) Int. Cl.: C12N 15/85, C12N 5/10

(54) **CELL PRODUCTION METHOD**

(30) Priority: 30.03.2018 JP 2018069241
(71) Applicant: Kyoto University, Kyoto-shi, Kyoto 606-8501 (JP); Takeda Pharmaceutical Company Limited, Osaka-shi, Osaka 541-0045, (JP)
(72) Inventor: YOSHIDA, Yoshinori, Kyoto-shi, Kyoto 606-8501 (JP); MIKI, Kenji, Kyoto-shi, Kyoto 606-8501 (JP); KAMACHI, Yasuharu, Fujisawa-shi, Kanagawa 251-0012 (JP)
(74) Representative: Jones, Nicholas Andrew
(86) International application number: PCT/JP2019/013518
(87) International publication number: WO 2019/189545

(57) **Abstract**

A production method comprising the step of forming an aggregate of cells under conditions that permit transfection of the cells with a substance in a non-cell-adhesive container is provided as a technique for producing cells having the desired substance introduced therein on a large scale at a commercial level.

## Description

### Technical Field

The present invention relates to a cell production method. More specifically, the present invention relates to a method for producing cells having the desired substance introduced therein.

### Background Art

The introduction of substances such as nucleic acids (e.g., DNA, and RNA such as mRNA and siRNA) to adherent cells has heretofore been performed by inoculating the cells to containers such as petri dishes and dishes, and then applying the substances and transfection reagents (e.g., liposome and polyamine) to the cells in a state adhering to the surface of the containers. Hence, it is difficult to produce cells having the desired substance introduced therein on a large scale at a commercial level, due to limitations to the surface areas of containers for cell adhesion.

A method is also known which involves introducing a substance to cells by inoculating adherent cells to a container such as a petri dish or a dish after adhesion of the substance and a transfection reagent to the surface of the container or coating of this surface therewith (so-called reverse transfection method) (Patent Literature 1). In this method, the substance is probably introduced into the cells during a process in which the inoculated cells adhere to the surface of the container. Hence, it is still difficult to produce cells having the desired substance introduced therein on a large scale at a commercial level, due to limitations to the surface areas of containers for cell adhesion.

### Citation List

### Patent Literature

Patent Literature 1: Japanese Translation of PCT International Application Publication No. 2008-532480
Patent Literature 2: International Publication No. WO 2015/141827

### Non Patent Literature

Non Patent Literature 1: Miki et al., "Efficient Detection and Purification of Cell Populations Using Synthetic MicroRNA Switches", Cell Stem Cell, 16, 1-13, 2015

### Summary of Invention

### Technical Problem

A main object of the present invention is to provide a technique for producing cells having the desired substance introduced therein on a large scale at a commercial level.

### Solution to Problem

In order to attain the object, the present invention provides the following [1] to [35].
[1] A method for producing cells having the desired substance introduced therein, the production method comprising the step of
   forming an aggregate of cells under conditions that permit transfection of the cells with the substance in a non-cell-adhesive container (step 1).
[2] The production method according to [1], wherein the cells are adherent cells.
[3] The production method according to [2], wherein the step 1 is the step of culturing the cells in the presence of a transfection reagent and the substance, and a microcarrier in the non-cell-adhesive container to form an aggregate comprising a cell having the substance introduced therein and the microcarrier.
[4] The production method according to [3], wherein the step 1 is performed under static culture of the cells.
[5] The production method according to [2], wherein the step 1 is the step of culturing the cells in the presence of a transfection reagent and the substance in the non-cell-adhesive container to form an aggregate comprising cells having the substance introduced therein.
[6] The production method according to [5], wherein the step 1 is performed under static culture of the cells.
[7] The production method according to any of [1] to [6], wherein the substance is a nucleic acid or a protein.
[8] The production method according to [7], wherein the nucleic acid is miRNA-responsive RNA comprising (i) a nucleic acid sequence specifically recognizing microRNA (miRNA) expressed in specific cells, and (ii) a nucleic acid sequence corresponding to a gene coding region of a desired protein (wherein the translation of the nucleic acid sequence (ii) into the protein is controlled by the nucleic acid sequence (i)).
[9] The production method according to any of [1] to [8], wherein the cells are cardiomyocytes.
[10] Cardiomyocytes obtained by a production method according to [9].
[11] A method for producing cells of interest, the production method comprising the steps of:
   forming an aggregate of cells under conditions that permit transfection of the cells with a desired substance in a non-cell-adhesive container (step 1); and
   sorting cells having the substance introduced therein on the basis of an intracellular function of the substance to select the cells of interest (step 2).
[12] The production method according to [11], wherein the cells are adherent cells.
[13] The production method according to [12], wherein the step 1 is the step of culturing the cells in the presence of a transfection reagent and the substance, and a microcarrier in the non-cell-adhesive container to form an aggregate comprising a cell having the substance introduced therein and the microcarrier.
[14] The production method according to [13], wherein the step 1 is performed under static culture of the cells.
[15] The production method according to [12], wherein the step 1 is the step of culturing the cells in the presence of a transfection reagent and the substance in the non-cell-adhesive container to form an aggregate comprising cells having the substance introduced therein.
[16] The production method according to [15], wherein the step 1 is performed under static culture of the cells.
[17] The production method according to any of [11] to [16], wherein the substance is a nucleic acid or a protein.
[18] The production method according to [17], wherein the nucleic acid is miRNA-responsive RNA comprising (i) a nucleic acid sequence specifically recognizing microRNA expressed in specific cells, and (ii) a nucleic acid sequence corresponding to a gene coding region of a desired protein (wherein the translation of the nucleic acid sequence (ii) into the protein is controlled by the nucleic acid sequence (i)).
[19] The production method according to [18], wherein
   the cells include cardiomyocytes and non-cardiomyocyte cells, the cells of interest are the cardiomyocytes, and in the step 2, cells having the miRNA-responsive RNA introduced therein are sorted on the basis of the presence or absence of translation into the protein to select the cardiomyocytes.
[20] The production method according to [18], wherein
   the cells are cardiomyocytes including ventricular myocytes, the cells of interest are the ventricular myocytes, and
   in the step 2, cardiomyocytes having the miRNA-responsive RNA introduced therein are sorted on the basis of the presence or absence of translation into the protein to select the ventricular myocytes.
[21] Cardiomyocytes obtained by a production method according to [19].
[22] Ventricular myocytes obtained by a production method according to [20].
[23] A method for introducing a desired substance to cells, the method comprising the step of forming an aggregate of cells under conditions that permit transfection of the cells with the substance in a non-cell-adhesive container.
[24] A method for producing cells of interest from pluripotent or multipotent stem cells, the production method comprising the steps of:
   inducing the differentiation of the pluripotent or multipotent stem cells into the cells of interest (step A) ;
   dispersing the cells thus induced by the differentiation (step B);
   introducing the cells thus dispersed into a non-cell-adhesive container to form an aggregate of the cells under conditions that permit transfection of the cells with a desired substance (step C); and
   sorting cells having the substance introduced therein on the basis of an intracellular function of the substance to select the cells of interest (step D).
[25] The production method according to [24], wherein the cells are adherent cells.
[26] The production method according to [25], wherein the step C is the step of culturing the cells in the presence of a transfection reagent and the substance, and a microcarrier in the non-cell-adhesive container to form an aggregate comprising a cell having the substance introduced therein and the microcarrier.
[27] The production method according to [26], wherein the step C is performed under static culture of the cells.
[28] The production method according to [27], wherein the step C is the step of culturing the cells in the presence of a transfection reagent and the substance in the non-cell-adhesive container to form an aggregate comprising cells having the substance introduced therein.
[29] The production method according to [28], wherein the step C is performed under static culture of the cells.
[30] The production method according to any of [24] to [29], wherein the substance is a nucleic acid or a protein.
[31] The production method according to [30], wherein the nucleic acid is miRNA-responsive RNA comprising (i) a nucleic acid sequence specifically recognizing microRNA expressed in specific cells, and (ii) a nucleic acid sequence corresponding to a gene coding region of a desired protein (wherein the translation of the nucleic acid sequence (ii) into the protein is controlled by the nucleic acid sequence (i)).
[32] The production method according to [31], wherein
   the cells of interest are cardiomyocytes, and in the step D, cells having the miRNA-responsive RNA introduced therein are sorted on the basis of the presence or absence of translation into the protein to select the cardiomyocytes.
[33] The production method according to [31], wherein
   the cells of interest are ventricular myocytes, and in the step D, cardiomyocytes having the miRNA-responsive RNA introduced therein are sorted on the basis of the presence or absence of translation into the protein to select the ventricular myocytes.
[34] Cardiomyocytes obtained by a production method according to [32].
[35] Ventricular myocytes obtained by a production method according to [33].

### Advantageous Effects of Invention

The present invention provides a technique for producing cells having the desired substance introduced therein on a large scale at a commercial level.

### Brief Description of Drawings

[Figure 1] Figure 1 is a photograph in which fluorescence attributed to EmGFP from cells having EmGFP introduced therein was confirmed in Example 1. The white portion actually depicts green fluorescence attributed to EmGFP.
[Figure 2] Figure 2 is a photograph in which fluorescence attributed to EmGFP from cells having EmGFP introduced therein was confirmed in Example 2. The white portion actually depicts green fluorescence attributed to EmGFP.

### Description of Embodiments

Hereinafter, suitable modes for carrying out the present invention will be described. The embodiments described below are given merely for illustrating typical embodiments of the present invention. The scope of the present invention should not be interpreted as being limited by these embodiments.

As used herein, the term "transfection" means the introduction of an arbitrary substance to the inside of cells. The inside of cells includes the inside of cytoplasms and the inside of nuclei.

As used herein, the term "desired substance" means a selected substance that is introduced to the inside of cells by transfection. The desired substance is not particularly limited as long as the desired substance is introducible to the inside of cells. Examples thereof include nucleic acids, proteins, and particles. A nucleic acid is preferred, and RNA is more referred.

The amount of the desired substance used may vary depending on the type of the substance, the type of the cells for use in introduction and transfection conditions, etc. For example, the amount of RNA used is 1 ng to 100 µg, preferably 100 ng to 5000 ng, per 1 × 10⁶ cells.

The phrase "conditions that permit transfection of cells with a substance" means conditions that may induce transfection. In the present invention, the "conditions that permit transfection of the cells with the substance" particularly specifically refers to "culturing the cells in the presence of a transfection reagent and the substance, and a microcarrier in a non-cell-adhesive container to form an aggregate comprising a cell having the substance introduced therein and the microcarrier" or "culturing the cells in the presence of a transfection reagent and the substance in a non-cell-adhesive container to form an aggregate comprising cells having the substance introduced therein".

The term "culture" or "culturing" means maintaining cells and/or allowing the cells to proliferate (grow) and/or differentiate out of tissue or the body, for example, in a dish, a petri dish, a flask, or a culture tank.

The term "adherent cells" means cells that are most suitably maintained or allowed to proliferate (grow) in a state adhering to solid layer surface serving as a scaffold. The adherent cells can be cells obtained directly from animal or human tissue (primary cells) or cells obtained by passaging these cells, and may be cells established as a line.

Examples of the cells obtained from animal or human tissue include myocytes including cardiomyocytes, and epithelial cells including pancreatic cells and hepatocytes.

Examples of the established cell line include mouse cell lines such as 3T3, NTCT and WEHI lines, hamster cell lines such as BHK (particularly, BHK21) and CHO lines, dog cell lines such as a MDCK line, pig cell lines such as a PK15 line, bovine cell lines such as a MDBK line, monkey cell lines such as Vero, LLC-MK2, FRHL2 and MA104 lines, and human cell lines such as MRC5, HEK293, PER.C6, Hela, ECV and A431 lines.

The term "pluripotency" means the ability to be able to differentiate into tissues and cells having various different shapes and functions and to be able to differentiate into cells of any lineage of the 3 germ layers. The term "pluripotency" is different from "totipotency", which is the ability to be able to differentiate into any tissue of the living body, including the placenta, in that pluripotent cells cannot differentiate into the placenta and therefore, do not have the ability to form an individual.

The term "multipotency" means the ability to be able to differentiate into plural and limited numbers of linages of cells. For example, mesenchymal stem cells, hematopoietic stem cells, neural stem cells are multipotent, but not pluripotent.

Examples of the "stem cells" include pluripotent stem cells.

The term "pluripotent stem cells" that may be used in the present invention refer to stem cells that can differentiate into tissues and cells having various different shapes and functions of the living body and have the ability to differentiate into cells of any lineage of the 3 germ layers (endoderm, mesoderm, and ectoderm). Examples thereof include, but are not particularly limited to, embryonic stem cells (ESCs), embryonic stem cells derived from cloned embryos obtained by nuclear transplantation, spermatogonial stem cells, embryonic germ cells, and induced pluripotent stem cells (herein also referred to as "iPSCs").

The term "multipotent stem cells" that may be used in the present invention refer to stem cells having the ability to be able to differentiate into plural and limited numbers of linages of cells. Examples of the "multipotent stem cells" that may be used in the present invention include dental pulp stem cells, oral mucosa-derived stem cells, hair follicle stem cells, and somatic stem cells derived from cultured fibroblasts or bone marrow stem cells. The pluripotent stem cells are preferably ESCs and iPSCs.

The term "induced pluripotent stem cells (iPSCs)" refer to cells that are obtained by reprograming mammalian somatic cells or undifferentiated stem cells by introducing particular factors (nuclear reprogramming factors). At present, there are various "induced pluripotent stem cells", and iPSCs established by Yamanaka, et al. by introducing the 4 factors Oct3/4, Sox2, Klf4, and c-Myc into murine fibroblasts (Takahashi K, Yamanaka S., Cell, (2006) 126: 663-676); iPSCs derived from human cells, established by introducing similar 4 factors into human fibroblasts (Takahashi K, Yamanaka S., et al. Cell, (2007) 131: 861-872.); Nanog-iPS cells established by sorting cells using expression of Nanog as an indicator after introduction of the 4 factors (Okita, K., Ichisaka, T., and Yamanaka, S. (2007). Nature 448, 313-317.); iPS cells produced by a method not using c-Myc (Nakagawa M, Yamanaka S., et al. Nature Biotechnology, (2008) 26, 101-106); iPS cells established by introducing 6 factors by a virus-free method (Okita K et al. Nat. Methods 2011 May; 8(5): 409-12, Okita K et al. Stem Cells. 31 (3) 458-66); and the like may be also used. Also, induced pluripotent stem cells established by introducing the 4 factors OCT3/4, SOX2, NANOG, and LIN28 by Thomson et al. (Yu J., Thomson JA. et al., Science (2007) 318: 1917-1920.); induced pluripotent stem cells produced by Daley et al. (Park IH, Daley GQ.et al., Nature (2007) 451: 141-146); induced pluripotent stem cells produced by Sakurada et al. (Japanese Unexamined Patent Application Publication No. 2008-307007) and the like may be used.

In addition, any of known induced pluripotent stem cells known in the art described in all published articles (for example, Shi Y., Ding S., et al., Cell Stem Cell, (2008) Vol 3, Issue 5, 568-574; Kim JB., Scholer HR., et al., Nature, (2008) 454, 646-650; Huangfu D., Melton, DA., et al., Nature Biotechnology, (2008) 26, No. 7, 795-797) or patents (for example, Japanese Unexamined Patent Application Publication No. 2008-307007, Japanese Unexamined Patent Application Publication No. 2008-283972, US2008-2336610, US2009-047263, WO2007-069666, WO2008-118220, WO2008-124133, WO2008-151058, WO2009-006930, WO2009-006997, WO2009-007852) may be used.

Available "induced pluripotent cells" include various iPSC lines established by NIH, Riken (Institute of Physical and Chemical Research), Kyoto University and the like. Examples of such human iPSC lines include HiPS-RIKEN-1A line, HiPS-RIKEN-2A line, HiPS-RIKEN-12A line, Nips-B2 line from Riken, and 253G1 line, 201B7 line, 409B2 line, 454E2 line, 606A1 line, 610B1 line, 648A1 line from Kyoto University. Alternatively, for example, cell lines of clinical grade provided from Kyoto University, Cellular Dynamics International and the like, and research and clinical cell lines prepared using these cell lines may be used.

Available "embryonic stem cells (ESCs)" include murine ESCs such as various murine ESC lines established by inGenious Targeting Laboratory, Riken (Institute of Physical and Chemical Research), and the like, and human ESCs such as various human ESC lines established by NIH, Riken, Kyoto University, and Cellartis. For example, CHB-1 to CHB-12 lines, RUES1 line, RUES2 line, and HUES1 to HUES28 lines from NIH, H1 line and H9 line from WiCell Research, and KhES-1 line, KhES-2 line, KhES-3 line, KhES-4 line, KhES-5 line, SSES1 line, SSES2 line, and SSES3 line from Riken can be used as the human ESC lines. Alternatively, for example, cell lines of clinical grade and research and clinical cell lines prepared using these cell lines may be used.

The term "comprise(s)" or "comprising" refers to inclusion of the element(s) following the word without limitations thereto. Thus, this suggests inclusion of the element(s) following the word, but does not suggest exclusion of any other element.

The phrase "consist(s) of" or "consisting of" means inclusion of all the element(s) following the phrase and limitation thereto. Accordingly, the phrase "consist(s) of" or "consisting of" indicates that the enumerated element(s) is required or essential and substantially no other elements exist. The phrase "consist(s) essentially of" or "consisting essentially of" means inclusion of any element following the phrase and limitation of other elements to those that do not affect the activity or effect of the enumerated element(s) specified in the disclosure. Accordingly, the phrase "consist(s) essentially of" or "consisting essentially of" indicates that the enumerated element(s) is required or essential, but other elements are optional and may exist or not exist depending on whether they affect the activity or effect of the enumerated element(s).

The cell production method according to the present invention is a method for producing cells having the desired substance introduced therein and comprises the following step 1.

Step 1: the step of forming an aggregate of cells under conditions that permit transfection of the cells with the substance in a non-cell-adhesive container.

The cell production method according to another aspect of the present invention is a method for producing cells of interest and comprises the following step 2 in addition to the step 1.

Step 2: the step of sorting cells having the substance introduced therein on the basis of an intracellular function of the substance to select the cells of interest.

The step 1 corresponds to the method for introducing a desired substance to cells according to the present invention.

The step 1 more specifically comprises any of the following step 1-1 and step 1-2.
Step 1-1: the step of culturing the cells in the presence of a transfection reagent and the substance, and a microcarrier in the non-cell-adhesive container to form an aggregate comprising a cell having the substance introduced therein and the microcarrier.
Step 1-2: the step of culturing the cells in the presence of a transfection reagent and the substance in the non-cell-adhesive container to form an aggregate comprising cells having the substance introduced therein.

As used herein, the cells of interest can be, for example, a mature cell population including a plurality of subtypes. A mature cardiomyocyte population as the mature cell population including a plurality of subtypes includes subtypes such as ventricular myocytes, atrial myocytes and pacemaker cells.

In the cell production method according to the present invention, the cells are not particularly limited as long as the cells are eukaryotic cells. The cells can be, for example, yeast cells, fungi cells, protist cells, plant cells, insect cells, amphibian cells, reptile cells, bird cells, non-human mammalian cells, or human cells. Examples of the non-human mammal include mice, rats, hamsters, guinea pigs, rabbits, dogs, cats, pigs, cattle, horses, sheep and monkeys. The cells can be cultured cells (*in vitro* and *ex vivo*)*.*

The cells are preferably adherent cells. Particularly, the cells are cells that have the ability to adhere to a microcarrier and may adhere to the microcarrier when cultured together with the microcarrier in a non-cell-adhesive container. Alternatively, the cells are cells that have the ability to aggregate and may form an aggregate when cultured in a non-cell-adhesive container.

The cells can be of one type or two or more types.

The substance to be introduced to the cells is not particularly limited and can be, for example, a nucleic acid, a protein or particles.

Examples of the nucleic acid include DNA, RNA and plasmids. These nucleic acids may contain a non-natural nucleoside.

Examples of the RNA particularly include miRNA-responsive RNA comprising (i) a nucleic acid sequence specifically recognizing microRNA (miRNA) expressed in specific cells, and (ii) a nucleic acid sequence corresponding to a gene coding region of a desired protein. In the miRNA-responsive RNA, the nucleic acid sequence (i) recognizes targeted miRNA and thereby controls the translation of the nucleic acid sequence (ii) into the protein. Specifically, the nucleic acid sequence (i) forms a duplex by binding to miRNA expressed in specific cells, and thereby inhibitorily controls (i.e., switches off) transcription and translation from the gene coding region of the nucleic acid sequence (ii) (which will be mentioned later in detail).

The plasmid can incorporate therein a gene to be expressed in the cells, a promoter for gene expression, and the like.

Examples of the protein include antibodies, enzymes and fluorescent proteins.

Examples of the particles include resin or metal microbeads, and colloid particles such as gold colloids and silver colloids. The microbeads may be modified with a compound or a polymer or may contain a compound or polymer, and can be prepared into, for example, affinity beads bound with an antibody, avidin/biotin or the like, fluorescent beads, or drug sustained-release beads. Examples of the compound or the drug include sensors, activators or inhibitors of intracellular reaction, acids and alkalis. The microbeads may be magnetic beads such as ferrite beads. The polymer can be a functional polymer that varies structurally in response to temperature or pH.

### Step 1: Aggregate formation step

In the step 1-1, the cells are cultured in the presence of a transfection reagent and the substance, and a microcarrier in the non-cell-adhesive container. The cells are preferably subjected in a dispersed state to culture and, if necessary, detached from the microcarrier and dispersed in a step prior to the step 1. The detachment treatment can adopt chemical treatment with an enzyme and a chelating agent, etc., physical treatment by pipetting, etc., and a combination thereof. A commercially available reagent (e.g., Liberase TM Research Grade, F. Hoffmann-La Roche, Ltd.; StemPro Accutase Cell Dissociation Reagent, Gibco/Thermo Fisher Scientific Inc.; and TrypLE Select CTS, Gibco/Thermo Fisher Scientific Inc.) can be used in the chemical treatment. The dispersion treatment can also be performed by an approach known in the art and can adopt, for example, chemical treatment with an enzyme and a chelating agent, etc., physical treatment by pipetting, etc., and a combination thereof. A commercially available reagent (e.g., Liberase TM Research Grade, F. Hoffmann-La Roche, Ltd.; StemPro Accutase Cell Dissociation Reagent, Gibco/Thermo Fisher Scientific Inc.; and TrypLE Select CTS, Gibco/Thermo Fisher Scientific Inc.) can be used in the chemical treatment.

In the culture, a non-cell-adhesive container is used for forming an aggregate comprising a cell and the microcarrier or an aggregate comprising cells alone. The non-cell-adhesive container that can be used is a container with its surface not artificially treated (e.g., by coating with extracellular matrix or the like) for the purpose of improving adhesiveness to cells. Alternatively, a container with its surface artificially treated (e.g., by coating with a hydrophobic molecule) for the purpose of reducing adhesiveness to cells may be used. The term "non-cell-adhesive" means that adherent cells do not adhere or rarely adhere to the container (e.g., less than 20%, preferably less than 10%, more preferably less than 1% cells based on the total number of cells inoculated to the container adhere to the container).

Examples of such a container include containers made of materials such as polycarbonate, polyethylene, polypropylene, Teflon(R), polyethylene terephthalate, polymethyl methacrylate, nylon 6,6, polyvinyl alcohol, cellulose, silicon, polystyrene, glass, polyacrylamide, polydimethylacrylamide, and stainless.

The container can be a microplate, a petri dish (dish), a cell culture flask (a stirring flask, a shaker flask, etc.), a cell culture bag, a roller bottle, a bioreactor or a culture tank, etc. and is appropriately selected according to a production scale (e.g., 1 mL to 2000 L). In the present invention, particularly, a large-capacity (e.g., 100 mL to 2000 L) container may be suitably used.

The transfection reagent can be a conventional reagent known in the art. For example, cationic liposomes (Lipofectamine MessengerMAX (Thermo Fisher Scientific Inc.), mRNAIn stem (LifeGene), etc.), lipids (HiPerFect Transfection Reagent (Qiagen N.V.), TransMessenger Transfection Reagent (Qiagen N.V.), Lipofectamine Stem reagents (Qiagen N.V.), Lipofectamine Stem reagents (Thermo Fisher Scientific Inc.), RiboJuice mRNA Transfection Kit (Merck KGaA), etc.), calcium phosphate (ProFection(R) Mammalian Transfection System (Promega Corp.), etc.), or polymers (jetPEI (Polyplus transfection), DEAE Dextran (GE Healthcare Bio-Sciences AB), etc.) may be used. Also, lipid nanoparticles (LNPs) containing a lipid molecule such as cationic lipid can be used as the transfection reagent. The transfection reagent can be appropriately selected according to the type of the cells or the type of the substance to be introduced to the cells.

The amount of the transfection reagent used may vary depending on the reagent, the cells and transfection conditions, etc. Typically, in the case of introducing 600 ng of mRNA to 2 × 10⁶ cells, 7.5 µL of Lipofectamine MessengerMAX is used.

The transfection can also be performed by a conventional method known in the art (e.g., electroporation and lipofection) instead of using the transfection reagent. For example, according to the electroporation, appropriate voltage is applied to a buffer solution containing cells with the applied substance, so that the substance passes through the cell membranes and is introduced into the cells. As another example, according to the lipofection, a complex of a lipid and the substance is applied to cells, so that the complex passes through the cell membranes to introduce the substance into the cells. As described above, the transfection approach according to the present invention is not particularly limited. For example, an approach described in Nature Protocols (K. Yusa et al., vol. 8, No. 10, 2013, 2061-2078), Nature Protocols (T. Sakuma et al., vol. 11, No. 1, 2016, 118-133), or Nature Protocols (B. Wefers et al., vol. 8, No. 12, 2013, 2355-2379) can be adopted.

The microcarrier has a surface to which cells may adhere, and means a carrier that can subject the cells to culture by suspending the microcarrier with the cells adhering thereto in a liquid medium. The microcarrier is not particularly limited by its material, shape and size, etc. as long as the carrier with the cells in a state adhering to the surface can be suspended in a liquid medium to culture the cells.

Examples of the material of the microcarrier include dextran, gelatin, collagen, polystyrene, polyethylene, polyacrylamide, glass, and cellulose.

Examples of the shape of the microcarrier include a spherical shape (beads) and a disk-like shape.

The size of the spherical microcarrier is, for example, 2 to 1000 µm, preferably 100 to 300 µm, in terms of diameter.

The microcarrier may be porous.

The number of microcarriers for use in cell culture is not particularly limited and is, for example, one microcarrier per ten cells.

The amount of the microcarrier used in cell culture is not particularly limited and is, for example, 0.1 g of the microcarrier per 1 × 10⁶ to 5 × 10⁷ cells, preferably 0.1 g of the microcarrier per 2 × 10⁷ to 3 × 10⁷ cells.

The microcarrier may be a commercially available product. For example, high-concentration Synthemax II microcarrier (Corning Inc.) may be used.

Adherent cells are statically cultured by the addition of the transfection reagent and the desired substance, and the microcarrier in the non-cell-adhesive container, so that the cells adhere to the microcarrier to form an aggregate. During this process, the substance is introduced to the cells.

In this context, the "aggregate" means an assembly comprising at least one cell and one microcarrier.

The size of the aggregate depends on the size of the microcarrier used and is not particularly limited.

The number of cells adhering to one microcarrier is not particularly limited and is, for example, 2 to 500, preferably 50 to 300.

The ratio of aggregated cells to the total number of the aggregated cells and unaggregated cells in the cultured cells is not particularly limited and is, for example, 10% or more, 20% or more, 30% or more, 40% or more, 50% or more, 60% or more, 70% or more, 80% or more, 90% or more, or 100%.

In the step 1-2, the cells are cultured in the presence of a transfection reagent and the substance without the use of a microcarrier.

Adherent cells are statically cultured by the addition of the transfection reagent and the substance in the non-cell-adhesive container, so that the cells form an aggregate even without the microcarrier. During this process, the substance is introduced to the cells.

In this context, the term "aggregate" means an assembly comprising at least two cells. Most of aggregates contain many cells adhering to one another.

The number of cells constituting one aggregate is not particularly limited and is, for example, 2 to 500, preferably 50 to 300.

The ratio of aggregated cells to the total number of the aggregated cells and unaggregated cells in the cultured cells is not particularly limited and is, for example, 10% or more, 20% or more, 30% or more, 40% or more, 50% or more, 60% or more, 70% or more, 80% or more, 90% or more, or 100%.

In the step 1 (step 1-1 and step 1-2), the cells may be transfected by culture with the substance in the presence or absence of the microcarrier without the use of the transfection reagent (e.g., electroporation).

The cells for aggregate formation is statically cultured for a period appropriate for the completion of transfection. The static culture period is not particularly limited, and can be, for example, on the order of 2 to 10 hours and is preferably on the order of 3 to 6 hours, particularly preferably 4 to 5 hours. The culture conditions are not particularly limited and can involve culture at approximately 37°C in the presence of 5% CO₂.

After the static culture, stirring culture may be performed in order to maintain the formed aggregate. The static culture period is not particularly limited, and can be, for example, on the order of 12 to 48 hours and is preferably on the order of 24 hours. The culture conditions are not particularly limited and can involve culture at approximately 37°C in the presence of 5% CO₂.

The stirring rate and time are appropriately set according to a cell density and the size of the culture container. Typically, the cells are left standing for 4 to 6 hours and then stirred at 25 rpm for 12 hours or longer. Excessive stirring or shaking places physical stress on the cells and also inhibits the maintenance of the aggregate. Thus, it is desirable to control the stirring rate so as to be able to uniformize medium components and the internal oxygen concentration of the medium and so as not to inhibit the maintenance of the aggregate.

A conventional medium known in the art can be used as the medium without particular limitations. For example, BME medium, BGJb medium, CMRL 1066 medium, Glasgow MEM medium, improved MEM (IMEM) medium, improved MDM (IMDM) medium, Medium 199 medium, Eagle MEM medium, αMEM medium, DMEM medium (high glucose or low glucose), DMEM/F12 medium, Ham's medium, RPMI 1640 medium, Fischer's medium, or a mixed medium thereof is used.

The amount of the medium used may vary depending on the cells or the culture conditions and may be appropriately determined by those skilled in the art.

The medium may be supplemented, if necessary, with an additive such as an amino acid, L-glutamine, GlutaMAX (product name), a non-essential amino acid, a vitamin, an antibiotic (e.g., penicillin, streptomycin, or a mixture thereof), an antimicrobial agent (e.g., amphotericin B), an antioxidant, pyruvic acid, a buffer, or inorganic salts on a cell or culture condition basis.

The amount of the additive used may vary depending on the cells or the culture conditions and may be appropriately determined by those skilled in the art.

The cell production method according to the present invention enables to introduce the desired substance to cells by forming an aggregate. Hence, unlike a conventional method using a petri dish or a dish, the cell production method according to the present invention is capable of producing cells having the desired substance introduced therein on a large scale at a commercial level without being limited by the surface area of a container for cell adhesion.

### Step 2: Sorting step

In the step 2, cells having the desired substance introduced therein are sorted on the basis of an intracellular function of the substance to select the cells of interest.

The sorting to select the cells of interest can be performed, for example, by detecting a marker protein or a marker gene introduced to the inside of the cells by transfection with the desired substance. The marker may be a positive selection marker or a negative selection marker. Preferably, the marker is a cell surface marker. Particularly, a cell surface-positive selection marker allows concentration, isolation, and/or detection of living cells. The marker protein can be detected by use of immunological assay, for example, ELISA, immunostaining, or flow cytometry, using an antibody specific for the marker protein. An antibody that binds to a specific amino acid sequence of the marker protein or a specific sugar chain linked to the marker protein, etc. can be used as the antibody specific for the marker protein. The marker gene can be detected by use of a method of amplifying and/or detecting nucleic acid known in the art, for example, RT-PCR, microarray, biochip, or RNAseq.

Alternatively, the sorting to select the cells of interest may be performed using, for example, introduced fluorescent beads or magnetic beads. Cell sorting by flow cytometry using fluorescent beads or magnetic beads is well known in the technical field.

When the desired substance is RNA, the step 2 may be based on a microRNA (miRNA) switch method. The miRNA switch method can be carried out, for example, according to a method described in WO2015/105172, Patent Literature 2 and Non Patent Literature 1 using the miRNA-responsive RNA mentioned above. The method described in Patent Literature 2 and Non Patent Literature 1 is a method of sorting a cell group including cardiomyocytes coexisting with cells other than cardiomyocytes to select the cardiomyocytes (which will be mentioned later in detail).

In one embodiment of the present invention, the "cells of interest" are ventricular myocytes.

In this embodiment, in the step 2, cardiomyocytes having the miRNA-responsive RNA introduced therein are sorted on the basis of the presence or absence of translation into the desired protein to select the ventricular myocytes.

As used herein, the phrase "translation into the desired protein is present" means that the protein is detectable by a method known in the art such as fluorescence detection, absorbance, Western blotting, or

### ELISA.

The present invention also provides a method for producing cells of interest from pluripotent or multipotent stem cells, the method comprising the following step A to step D.
Step A: the step of inducing the differentiation of the pluripotent or multipotent stem cells into the cells of interest.
Step B: the step of dispersing the cells thus differentiation-induced.
Step C: the step of introducing the thus dispersed cells into a non-cell-adhesive container to form an aggregate of the cells under conditions that permit transfection of the cells with a desired substance.
Step D: the step of sorting cells having the substance introduced therein on the basis of an intracellular function of the substance to select the cells of interest.

Examples of the cells of interest differentiation-induced from the pluripotent or multipotent stem cells include, but are not particularly limited to, cardiomyocytes (including ventricular myocytes, atrial myocytes, and pacemaker cells), pancreatic progenitor cells, neural progenitor cells, hepatocytes and vascular endothelial cells. The induction of differentiation into these cells can be performed by the application of conventional conditions known in the art.

The method for producing cells of interest from pluripotent or multipotent stem cells according to the present invention can be applied to, for example, the separation of cardiomyocytes differentiation-induced from the pluripotent or multipotent stem cells, from non-cardiomyocyte cells (including undifferentiated pluripotent or multipotent stem cells), the separation of pancreatic progenitor cells differentiation-induced from the pluripotent or multipotent stem cells, from non-pancreatic-progenitor cells, the separation of neural progenitor cells differentiation-induced from the pluripotent or multipotent stem cells, from non-neural-progenitor cells, the separation of hepatocytes differentiation-induced from the pluripotent or multipotent stem cells, from non-hepatocyte cells, or the separation of vascular endothelial cells differentiation-induced from the pluripotent or multipotent stem cells, from non-vascular-endothelial cells.

Hereinafter, the method for producing cells of interest from pluripotent or multipotent stem cells according to the present invention will be specifically described with reference to an exemplary method of inducing cardiomyocytes from the pluripotent or multipotent stem cells, and sorting the cardiomyocytes by use of the miRNA switch method to select, particularly, ventricular myocytes.

### Step A: Differentiation induction step

In the step A, the differentiation of the pluripotent or multipotent stem cells into cardiomyocytes is induced.

In the present invention, the "cardiomyocytes" mean cells expressing at least cardiac troponin (cTnT) or αMHC. Examples of the cTnT include human cTnT represented by NCBI accession No. NM_000364, and mouse cTnT represented by NCBI accession No. NM_001130174. Examples of the αMHC include human αMHC represented by NCBI accession No. NM_002471, and mouse αMHC represented by NCBI accession No. NM_001164171.

The induction of differentiation into cardiomyocytes can adopt, for example, a method described in Patent Literature 2 and Non Patent Literature 1. Alternatively, the cardiomyocytes can be produced from the pluripotent or multipotent stem cells by, for example, a method reported by Laflamme MA et al. (Laflamme MA &Murry CE, Nature 2011, Review). Other examples of the method include, but are not particularly limited to, a method of producing cardiomyocytes by forming a cell mass (embryoid body) from induced pluripotent stem cells by suspension culture, a method of producing cardiomyocytes in the presence of a substance inhibiting BMP signal transduction (WO2005/033298), a method of producing cardiomyocytes by adding activin A and BMP in order (WO2007/002136), a method of producing cardiomyocytes in the presence of a substance producing the activation of the canonical Wnt signaling pathway (WO2007/126077) and a method of isolating Flk/KDR-positive cells from induced pluripotent stem cells, and producing cardiomyocytes in the presence of cyclosporin A (WO2009/118928).

### Step B: Dispersion step

In the step B, the cardiomyocytes thus differentiation-induced are dispersed.

The dispersion treatment can be performed by an approach known in the art and can adopt, for example, chemical treatment with an enzyme and a chelating agent, etc., physical treatment by pipetting, etc., and a combination thereof. A commercially available reagent (e.g., Liberase TM Research Grade, F. Hoffmann-La Roche, Ltd.; StemPro Accutase Cell Dissociation Reagent, Gibco/Thermo Fisher Scientific Inc.; and TrypLE Select CTS, Gibco/Thermo Fisher Scientific Inc.) can be used in the chemical treatment.

### Step C: Aggregate formation step

In the step C, an aggregate of cells is formed under conditions that permit transfection of the cardiomyocytes with microRNA (miRNA)-responsive RNA in a non-cell-adhesive container. Specific procedures of the step C are the same as in the step 1.

The miRNA-responsive RNA comprises (i) a nucleic acid sequence specifically recognizing miRNA specifically expressed in ventricular myocytes, and (ii) a nucleic acid sequence corresponding to a gene coding region of a desired protein, the nucleic acid sequence (ii) being functionally linked to the nucleic acid sequence (i). The translation of the nucleic acid sequence (ii) into the protein is controlled by the nucleic acid sequence (i).

The phrase "translation of the nucleic acid corresponding to the gene coding region of the nucleic acid sequence (ii) into the protein is controlled by the nucleic acid sequence (i)" means that, in the presence of miRNA specifically expressed in ventricular myocytes, the translation of the nucleic acid corresponding to the gene coding region into the protein is controlled according to the abundance thereof. More preferably, miRNA specifically expressed in ventricular myocytes forms a duplex by binding to the nucleic acid sequence (i), and thereby inhibits transcription and translation from the gene coding region of the nucleic acid sequence (ii) (off-switch miRNA-responsive RNA).

The functional linking of the nucleic acid sequence (ii) to the nucleic acid sequence (i) means that at least one nucleic acid sequence (i) is contained in 5' UTR, 3' UTR, and/or the open reading frame of the gene coding region of the nucleic acid sequence (ii).

The phrase "microRNA (miRNA) specifically expressed in ventricular myocytes" is not particularly limited as long as the miRNA is highly expressed in ventricular myocytes compared with cells other than ventricular myocytes. The miRNA can be expressed at least 10%, at least 20%, at least 30%, at least 40%, at least 50%, at least 60%, at least 70%, at least 80%, or at least 90% more highly in cardiomyocytes compared with cells other than ventricular myocytes, though the miRNA is not limited thereto. Such miRNA can be appropriately selected from miRNA registered in information of a database (e.g., http://www.mirbase.org/ or http://www.microrna.org/) and/or miRNA described in literature information described in the database.

The phrase "specifically recognizing miRNA specifically expressed in ventricular myocytes" regarding the nucleic acid sequence (i) means that the miRNA is present, in the form of RNA-induced silencing complex (RISC) formed through interaction with a plurality of predetermined proteins, on the nucleic acid sequence (i).

The phrase "gene coding region of a protein" regarding the nucleic acid sequence (ii) is a nucleic acid gene encoding a protein that permits sorting of the ventricular myocytes through intracellular translation.

As one example, the "gene" can be a marker gene. The "marker gene" is a gene encoding a protein that functions as a marker through intracellular translation and permits sorting of the ventricular myocytes.

Examples of the protein that may function as a marker through intracellular translation can include fluorescent proteins and apoptosis-inducing proteins. Various molecules known in the art can be used as the fluorescent proteins. Examples of the apoptosis-inducing protein include, but are not limited to, IKB, Smac/DIABLO, ICE, HtrA2/OMI, AIF, endonuclease G, Bax, Bak, Noxa, Hrk (harakiri), Mtd, Bim, Bad, Bid, PUMA, activated caspase-3, Fas, and Tk.

On-switch artificial RNA (miRNA switch; synthetic mRNA) composed of a sequence (miRNA target sites) recognizing miRNA (e.g., miR-208b-3p (SEQ ID NO: 1)) specific for ventricular myocytes, and a gene sequence of a fluorescent protein in combination can be used as the miRNA-responsive RNA. When this artificial RNA is introduced to cardiomyocytes, the fluorescent protein is expressed (switched on) by the binding of miRNA to the artificial RNA in ventricular myocytes where the miRNA is present. On the other hand, the fluorescent protein is not expressed in cells, other than ventricular myocytes, where the miRNA is absent, because the artificial RNA does not bind to the miRNA.

Alternatively, off-switch artificial RNA composed of a sequence recognizing miRNA specific for ventricular myocytes, and the sequence of an apoptosis-inducing gene (e.g., Bim) in combination may be used as the miRNA-responsive RNA. When this artificial RNA is introduced to cells, the expression of the gene causing apoptosis is suppressed (switched off) by the binding of the artificial RNA to miRNA in cardiomyocytes where the miRNA is present. On the other hand, the gene causing apoptosis is expressed to cause apoptosis in cells, other than ventricular myocytes, where the miRNA is absent, because the artificial RNA does not bind to the miRNA.

### Step D: Sorting step

In the step D, cardiomyocytes having the miRNA-responsive RNA introduced therein are sorted on the basis of an intra-cardiomyocyte function of the miRNA-responsive RNA to select the ventricular myocytes.

In the case of using the above-described on-switch artificial RNA composed of a sequence recognizing miRNA specific for ventricular myocytes, and a gene sequence of a fluorescent protein in combination, the ventricular myocytes which are positive for the expression of the fluorescent protein or have a predetermined value or higher of an expression level thereof are separated, using a cell sorter or the like, from cells, other than ventricular myocytes, which are negative for the expression of the fluorescent protein or have less than the predetermined value of the expression level.

In the case of using the above-described off-switch artificial RNA composed of a sequence recognizing miRNA specific for ventricular myocytes, and the sequence of an apoptosis-inducing gene in combination, the ventricular myocytes can be purified without sorting using equipment, because the cells other than ventricular myocytes undergo apoptosis.

The cell production method according to the present invention can introduce the substance to cells by forming an aggregate of the cells in the step C. Hence, the cell production method according to the present invention can produce cells having the desired substance introduced therein on a large scale at a commercial level without being limited by the surface area of a container for cell adhesion, and is capable of producing the differentiated cells of interest on a large scale at a commercial level in the step D.

### Examples

### [Example 1: Production of cardiomyocytes from iPSCs - 1]

In this Example, cardiomyocytes induced from iPSCs were stirring-cultured in the presence of a transfection reagent and an RNA for fluorescent protein expression (EmGFP mRNA) to form an aggregate, through which the RNA was introduced to the cardiomyocytes.

### (1) Induction of cardiomyocytes

iPSCs (Ff-I14s04, obtained from Center for iPS Cell Research and Application, Kyoto University) maintained and cultured in Essential 8 medium (Gibco/Thermo Fisher Scientific Inc.) using a 10 cm dish coated with Synthemax II (Corning Inc.) were inoculated at 1.5 × 10⁶ cells/mL to RPMI-1640 medium (Gibco/Thermo Fisher Scientific Inc.) in a 30 mL bioreactor. The RPMI-1640 medium was supplemented with 10 µmol/L Y-27632 (Wako Pure Chemical Industries, Ltd.) and contained 0.55 g (converted to a surface area of 198 cm²) of a microcarrier (high-concentration Synthemax II microcarrier, Corning Inc.).

After culture at 37°C for 2 days at a low oxygen concentration (5%) in an incubator, differentiation was induced for 21 days using RPMI-1640 medium as a basal medium.

### (2) RNA transfection

### (Preparation of RNA)

EmGFP mRNA, and miRNA-responsive off-switch mRNA consisting of miR-208a-3p and tagBFP were prepared by the following method based on DNA sequences (SEQ ID NO: 2 and SEQ ID NO: 3) corresponding to these mRNAs.

Each mRNA was prepared using MEGAscript T7 kit (Thermo Fisher Scientific Inc.) according to the protocol described in Warren L, et al., Cell Stem Cell. 7 (5): 618-30 (2010). In this reaction, pseudouridine-5'-triphosphate and methylcytidine-5'-triphosphate (TriLink BioTechnologies, Inc.) were used instead of uridine triphosphate and cytidine triphosphate, respectively. Before IVT (mRNA synthesis) reaction, guanidine-5'-triphosphate was diluted 5 times with Anti Reverse cap Analog (TriLink Biotechnologies, Inc.). The reaction mixture was incubated at 37°C for 5 hours. After addition of TURBO DNase (Thermo Fisher Scientific, Inc.), the mixture was further incubated at 37°C for 30 minutes. The obtained mRNA was purified through FavorPrep Blood / Cultured Cells total RNA extraction column (Favorgen Biotech Corp.) and incubated at 37°C for 30 minutes using Antarctic phosphatase (New England BioLabs Inc.). Then, the mRNA was further purified using RNeasy Mini Elute Cleanup Kit (Qiagen N.V.).

### (Transfection with mRNA)

After recovery of the cells (including cells on the microcarrier and cells dissociated from the microcarrier) induced by the differentiation, the detachment of the cells from the microcarrier and the dispersion of the cells were performed by the following procedures.

The cells were treated with IMDM (Iscove's Modified Dulbecco's Medium, supplemented with 1% DNase) containing Liberase (Liberase TM Research Grade, F. Hoffmann-La Roche, Ltd.) for 40 minutes.

Next, the cells were treated with Accutase (StemPro Accutase Cell Dissociation Reagent, Gibco/Thermo Fisher Scientific Inc.) for 10 minutes.

Finally, the cells were pipetted.

The cell solution thus pipetted was passed through a cell strainer (Sterile Cell Strainer 40 µm, Thermo Fisher Scientific Inc.) to recover single cells.

After removal of a supernatant by centrifugation, the cells were suspended at 2 × 10⁶ cells/mL in RPMI-1640 medium (supplemented with B27 Supplement 1/50 (vol/vol) and supplemented with 10 µmol/L Y-27632). 125 µL of Opti-MEM medium (Gibco/Thermo Fisher Scientific Inc.) as well as 7.5 µL of Lipofectamine MessengerMAX (Invitrogen Corp.), 3 µL of 100 ng/µL EmGFP mRNA, and 3 µL of 100 ng/µL miRNA-responsive off-switch mRNA consisting of miR-208a-3p and tagBFP were mixed per 2 × 10⁶ cells/mL and further mixed with the cell suspension. Immediately thereafter, the mixture was transferred to a 30 mL bioreactor (30 mL Single Use Bioreactor, Biott Corp.).

The cells were statically cultured at 37°C for 4 hours in an ordinary oxygen concentration (21%) in an incubator. Then, RPMI-1640 medium (supplemented with B27 Supplement 1/50 (vol/vol) and supplemented with 10 µmol/L Y-27632) was added to the 30 mL bioreactor such that the amount of the culture solution was 30 mL. Stirring culture was started at 25 rpm.

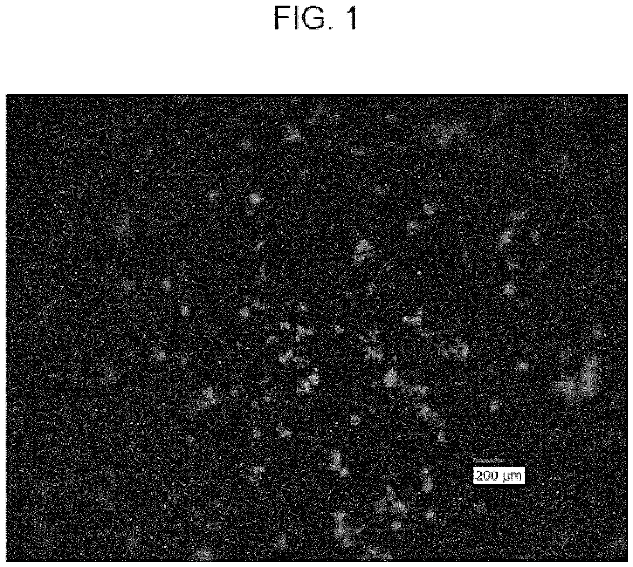

On the next day, cell aggregates were recovered by the centrifugation of the culture solution, treated with Accutase (StemPro Accutase Cell Dissociation Reagent, Gibco/Thermo Fisher Scientific Inc.) for 20 minutes, and then pipetted. RPMI-1640 medium (supplemented with 10 µg/mL bovine pancreas DNase) was added. The cells were further recovered by centrifugation, suspended in D-PBS(-) solution (Wako Pure Chemical Industries, Ltd.), and then centrifuged again to recover cells. To the recovered cells, 0.5% BSA/PBS solution (BSA (Wako Pure Chemical Industries, Ltd., 30 w/v% Albumin Solution, from Bovine Serum, Fatty Acid Free) added to PBS at 1/60 (vol/vol)) was added. Single cells were obtained by pipetting. Cells emitting fluorescence attributed to EmGFP were confirmed by use of flow cytometry (FACS Aria Fusion) (Figure 1).

Further, tag-BFP is also applied to flow cytometry, and a fluorescence emission ratio between EmGFP and tag-BFP is determined so that the cells can be sorted to select cardiomyocytes as cells with a reduced tag-BFP/EmGFP ratio.

### [Example 2: Production of cardiomyocytes from iPSCs - 2]

In this Example, cardiomyocytes induced from iPSCs were stirring-cultured in the presence of a transfection reagent, RNA for fluorescent protein expression (EmGFP mRNA) and a microcarrier to form an aggregate, through which the RNA was introduced to the cardiomyocytes.

### (1) Induction of cardiomyocytes

iPSCs (Ff-I14s04, obtained from Center for iPS Cell Research and Application, Kyoto University) maintained and cultured in Essential 8 medium (Gibco/Thermo Fisher Scientific Inc.) using a 10 cm dish coated with Synthemax II (Corning Inc.) were inoculated at 4 × 10⁵ cells/mL to RPMI-1640 medium (Gibco/Thermo Fisher Scientific Inc.) in a 30 mL bioreactor. The RPMI-1640 medium was supplemented with 10 µmol/L Y-27632 (Wako Pure Chemical Industries, Ltd.) and contained 0.55 g (converted to a surface area of 198 cm²) of a microcarrier (high-concentration Synthemax II microcarrier, Corning Inc.).

After culture at 37°C for 4 days at a low oxygen concentration (5%) in an incubator, differentiation was induced for 35 days using RPMI-1640 medium as a basal medium.

### (2) RNA transfection

### (Preparation of RNA)

100 ng/µL EmGFP mRNA, and 100 ng/µL miRNA-responsive off-switch mRNA consisting of miR-208a-3p and tagBFP were prepared in the same way as in Example 1.

### (Transfection with mRNA)

After recovery of the differentiation-induced cells (cells on the microcarrier and cells dissociated from the microcarrier), the detachment of the cells from the microcarrier and the dispersion of the cells were performed by the following procedures.

The cells were treated with IMDM (Iscove's Modified Dulbecco's Medium, supplemented with 1% DNase) containing Liberase (Liberase TM Research Grade, F. Hoffmann-La Roche, Ltd.) for 40 minutes.

Next, the cells were treated with TrypLE select (TrypLE Select CTS, Gibco/Thermo Fisher Scientific Inc.) for 10 minutes.

Finally, the cells were pipetted.

The cell solution thus pipetted was passed through a cell strainer (Sterile Cell Strainer 40 µm, Thermo Fisher Scientific Inc.) to recover single cells.

After removal of a supernatant by centrifugation, the cells were suspended in RPMI-1640 medium (supplemented with B27 Supplement 1/50 (vol/vol) and supplemented with 10 µmol/L Y-27632). The cell suspension (the number of cells: 18.2 × 10⁶) was mixed with 18 µL of mRNAIn stem (LifeGene), 27 µL of 100 ng/µL EmGFP mRNA, and 27 µL of 100 ng/µL miRNA-responsive off-switch mRNA consisting of miR-208a-3p and tagBFP. Immediately thereafter, the mixture was transferred to a 30 mL bioreactor (30 mL Single Use Bioreactor, Biott Corp.) containing 0.1g of a microcarrier (high-concentration Synthemax II microcarrier).

The cells were statically cultured at 37°C for 5 hours in an ordinary oxygen concentration (21%) in an incubator.

Then, RPMI-1640 medium (supplemented with B27 Supplement 1/50 (vol/vol) and supplemented with 10 µmol/L Y-27632) was added to the 30 mL bioreactor such that the amount of the culture solution was 30 mL. Stirring culture was started at 25 rpm.

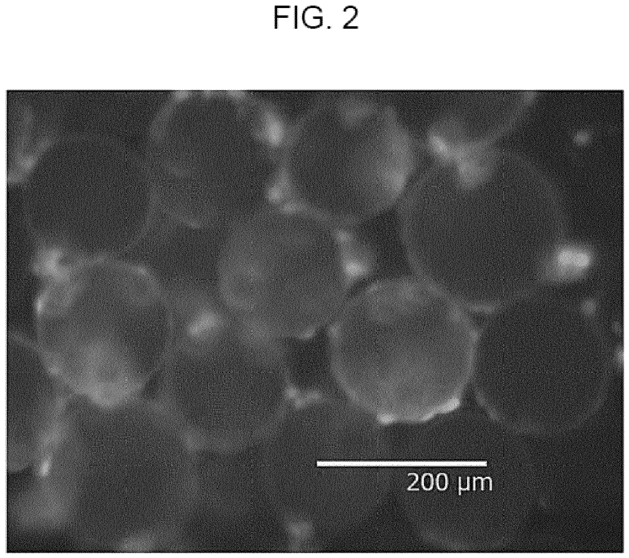

On the next day, the obtained cell aggregates were treated with Accutase (StemPro Accutase Cell Dissociation Reagent, Gibco/Thermo Fisher Scientific Inc.) in accordance with Example 1, and then pipetted. Single cells were recovered through a cell strainer (Sterile Cell Strainer 40 µm, Thermo Fisher Scientific Inc.). Cells emitting fluorescence attributed to EmGFP were confirmed by use of flow cytometry (FACS Aria Fusion) (Figure 2).

Further, tag-BFP is also applied to flow cytometry, and a fluorescence emission ratio between EmGFP and tag-BFP is determined so that the cells can be sorted to select cardiomyocytes as cells with a reduced tag-BFP/EmGFP ratio.

### Industrial Applicability

The present invention is useful for producing cells having the desired substance introduced therein on a large scale at a commercial level.

### Free Text of Sequence Listing

SEQ ID NO: 1: RNA sequence corresponding to human miR-208b-3p
SEQ ID NO: 2: DNA sequence corresponding to EmGFP mRNA
SEQ ID NO: 3: DNA sequence corresponding to miRNA-responsive off-switch mRNA consisting of miR-208a-3p and tagBFP

## Claims

1. A method for producing cells having the desired substance introduced therein, the production method comprising the step of
forming an aggregate of cells under conditions that permit transfection of the cells with the substance in a non-cell-adhesive container.

2. The production method according to claim 1, wherein the cells are adherent cells.

3. The production method according to claim 2, wherein the step is the step of culturing the cells in the presence of a transfection reagent and the substance, and a microcarrier in the non-cell-adhesive container to form an aggregate comprising a cell having the substance introduced therein and the microcarrier.

4. The production method according to claim 2, wherein the step is the step of culturing the cells in the presence of a transfection reagent and the substance in the non-cell-adhesive container to form an aggregate comprising cells having the substance introduced therein.

5. The production method according to claim 3 or 4, wherein the step is performed under static culture of the cells.

6. The production method according to any one of claims 1 to 5, wherein the substance is a nucleic acid or a protein.

7. The production method according to any one of claims 1 to 6, wherein the cells are cardiomyocytes.

8. A method for producing cells of interest, the production method comprising the steps of:
forming an aggregate of cells under conditions that permit transfection of the cells with a desired substance in a non-cell-adhesive container; and
sorting cells having the substance introduced therein on the basis of an intracellular function of the substance to select the cells of interest.

9. Ventricular myocytes obtained by the production method according to claim 8.


SEQUENCE LISTING

<110>  KYOTO UNIVERSITY
       TAKEDA PHARMACEUTICAL COMPANY LIMITED

<120>  METHOD FOR PRODUCING CELLS

<130>  PT38-9031WO

<150>  JP2018-069241
<151>  2018-03-30

<160>  3

<170>  PatentIn version 3.5

<210>  1
<211>  22
<212>  RNA
<213>  Homo sapiens

<400>  1
auaagacgaa caaaagguuu gu                                                22


<210>  2
<211>  1016
<212>  DNA
<213>  Artificial Sequence

<220>
<223>  EmGFP Sequence

<400>  2
cagtgaattg taatacgact cactataggg cgaattaaga gagaaaagaa gagtaagaag        60

aaatataaga caccggtcgc caccatggtg agcaagggcg aggagctgtt caccggggtg       120

gtgcccatcc tggtcgagct ggacggcgac gtaaacggcc acaagttcag cgtgtccggc       180

gagggcgagg gcgatgccac ctacggcaag ctgaccctga agttcatctg caccaccggc       240

aagctgcccg tgccctggcc caccctcgtg accaccttca cctacggcgt gcagtgcttc       300

gcccgctacc ccgaccacat gaagcagcac gacttcttca agtccgccat gcccgaaggc       360

tacgtccagg agcgcaccat cttcttcaag gacgacggca actacaagac ccgcgccgag       420

gtgaagttcg agggcgacac cctggtgaac cgcatcgagc tgaagggcat cgacttcaag       480

gaggacggca acatcctggg gcacaagctg gagtacaact acaacagcca caaggtctat       540

atcaccgccg acaagcagaa gaacggcatc aaggtgaact tcaagacccg ccacaacatc       600

gaggacggca gcgtgcagct cgccgaccac taccagcaga acacccccat cggcgacggc       660

cccgtgctgc tgcccgacaa ccactacctg agcacccagt ccgccctgag caaagacccc       720

aacgagaagc gcgatcacat ggtcctgctg gagttcgtga ccgccgccgg gatcactctc       780

ggcatggacg agctgtacaa gtaatctaga ccttctgcgg ggcttgcctt ctggccatgc       840

ccttcttctc tcccttgcac ctgtacctct tggtctttga ataaagcctg agtaggaaaa       900

aaaaaaaaaa aaaaaaaaaa aaaaaaaaaa aaaaaaaaaa aaaaaaaaaa aaaaaaaaaa 960

aaaaaaaaaa aaaaaaaaaa aaaaaaaaaa aaaaaaaaaa aaaaaaaaaa aaaaaa 1016

<210> 3
<211> 991
<212> DNA
<213> Artificial Sequence

<220>
<223> Off-switch mRNA sequence

<400> 3
gctaatacga ctcactatag gttccttaat cgcggatcca caagcttttt gctcgtctta 60

tagatcacac cggtcgccac catgagcgag ctgattaagg agaacatgca catgaagctg 120

tacatggagg gcaccgtgga caaccatcac ttcaagtgca catccgaggg cgaaggcaag 180

ccctacgagg gcacccagac catgagaatc aaggtggtcg agggcggccc tctccccttc 240

gccttcgaca tcctggctac tagcttcctc tacggcagca agaccttcat caaccacacc 300

cagggcatcc ccgacttctt caagcagtcc ttccctgagg gcttcacatg ggagagagtc 360

accacatacg aagacggggg cgtgctgacc gctacccagg acaccagcct ccaggacggc 420

tgcctcatct acaacgtcaa gatcagaggg gtgaacttca catccaacgg ccctgtgatg 480

cagaagaaaa cactcggctg ggaggccttc accgagacgc tgtaccccgc tgacggcggc 540

ctggaaggca gaaacgacat ggccctgaag ctcgtgggcg ggagccatct gatcgcaaac 600

atcaagacca catatagatc caagaaaccc gctaagaacc tcaagatgcc tggcgtctac 660

tatgtggact acagactgga aagaatcaag gaggccaaca acgagaccta cgtcgagcag 720

cacgaggtgg cagtggccag atactgcgac ctccctagca aactggggca caagcttaat 780

ctagaccttc tgcggggctt gccttctggc catgcccttc ttctctccct tgcacctgta 840

cctcttggtc tttgaataaa gcctgagtag gaaaaaaaaa aaaaaaaaaa aaaaaaaaaa 900

aaaaaaaaaa aaaaaaaaaa aaaaaaaaaa aaaaaaaaaa aaaaaaaaaa aaaaaaaaaa 960

aaaaaaaaaa aaaaaaaaaa aaaaaaaaaa a 991